# EUROPEAN PATENT APPLICATION

(11) **EP 4 481 751 A1**
(43) Date of publication of application: **25.12.2024**
(21) Application number: 23180212.5
(22) Date of filing: 20.06.2023
(51) Int. Cl.: G16H 10/60, G16H 40/20, G16H 40/63

(54) **CONTROL OF A USER INTERFACE**

(71) Applicant: Koninklijke Philips N.V., 5656 AG Eindhoven (NL)
(72) Inventor: HUIJBREGTS, Laurentia Johanna, Eindhoven (NL); VAN LIESHOUT, Ron Martinus Laurentius, Eindhoven (NL); BULUT, Murtaza, 5656AG Eindhoven (NL); HIWALE, Sujitkumar, 5656AG Eindhoven (NL); BOUTS, Mark Jacobus Rosalie Joseph, 5656AG Eindhoven (NL); LOPEZ, Benjamin, 5656AG Eindhoven (NL)
(74) Representative: Philips Intellectual Property & Standards

(57) **Abstract**

A mechanism for displaying timeline data and pre-timeline data of a subject. A first portion of a user interface is controlled to display the timeline data of one or more first parameters. A second portion of the user interface is controlled to display the pre-timeline data of one or more second parameters. The amount of time between the earliest value of the pre-timeline data and first point in time, per unit space of the second portion, is less than the amount of time between the first and second points in time, per unit space of the first portion.

## Description

### FIELD OF THE INVENTION

The present invention relates to user interfaces, and in particular to controlling a user interface to provide information about a subject.

### BACKGROUND OF THE INVENTION

In the medical field, there are a wide variety of graphical user interfaces for providing a clinician or other individual with information about a (medical) subject. Accordingly, approaches for controlling a user interface, such as a screen or display, to provide such information are known.

One area of interest for a clinician or individual is to review information about a period of time, e.g., information pertaining to a period of time between a first point in time and a second point in time. Such information may, for instance, comprise values of parameters monitored during the period of time, based on samples extracted during that period of time or for parameters performed on (or provided to) the subject during that period of time.

In general, parameters of a subject can be divided into at least three groups. A first group includes physiological parameters than can be continuously monitored, such as heart rate, mean arterial pressure (MAP), central venous pressure (CVP), cardiac output, respiratory rate, SpO2 and so on. A second group includes parameters that cannot be continuously monitored, such as urine output, lactate level, hemoglobin level and so on. A third group includes intervention parameters, being indicators of a time and/or detail on any interventions that were performed on the subject. Examples of intervention parameters include information regarding times and/or amounts of: noradrenaline, crystalloids, trombos (thrombocytes), plasma, packed cells, milrinone, and dobutamine. Other suitable examples will be readily apparent to the skilled person.

There is an ongoing demand to provide, at a user interface, suitable information that enables a clinician to make an accurate clinical decision. There is therefore a need to provide a method of controlling a user interface that allows information about a subject to be reviewed by a clinician or other individual.

### SUMMARY OF THE INVENTION

The invention is defined by the claims.

According to examples in accordance with an aspect of the invention, there is provided a computer-implemented method for controlling a user interface.

The computer-implemented method comprises: obtaining, for one or more first parameters of the subject, timeline data containing any values of the respective first parameter between a first point in time and a second, later point in time; obtaining, for one or more second parameters of the subject, pre-timeline data containing one or more values of the respective second parameter, each value of each second parameter being a value at a point in time before the first point in time;

The computer-implemented method also comprises controlling a first portion of the user interface to provide for each first parameter, a first visual representation of the timeline data; and controlling a second portion of the user interface to provide, for each second parameter, a second visual representation identifying at least the presence of any pre-timeline data for that second parameter.

The quotient produced by dividing the amount of space occupied by the first portion by a first length of time, being the length of time between the first point in time and the second point in time, defines a first quotient. The quotient produced by dividing the amount of space occupied by the second portion by a second length of time, being the length of time between the furthest point in time of any value of any second parameter to the first point in time and the first point in time, defines a second quotient. The first quotient is larger than the second quotient.

Embodiments provides a technique for displaying two sets of information. The first set of information is displayed in a first portion of a user interface, with a second set of information being displayed in a second portion of the same user interface. The first portion represents less time per unit space than the second portion. Thus, the total length of time represented by each unit space of the first portion is less than the total length of time represented by each unit space of the second portion.

The proposed approach provides a method for providing an individual, such as a clinician, with relevant information for making a clinical decision whilst maintaining an overview of the presence of other information that may be relevant for making the clinical decision. In particular, the proposed approach resolves conflicting requirements between displaying (in an enlarged area) clinical information within a particular period of time (to aid the individual in making the clinical decision) and maintaining an indicator of the presence of second information.

The proposed approach thereby assists a user in performing a technical task, e.g., of making a clinical decision, by means of a continued human-machine interaction process.

The one or more second parameters of the subject may contain only the first parameters of the subject; a subset of the first parameters of the subject; and/or one or more statistical properties of one or more of the first parameters of the subject. This improves the contextual relevance of the second portion for aiding an individual in making a clinical decision. In particular, the value(s) for the second parameters facilitates improved understanding of any changes in the condition of the subject or the like.

Preferably, the first portion of the user interface is no less than 3 times larger than the second portion of the user interface.

In some examples, the first and second portions occupy a same height of the user interface but different widths of the user interface.

In some examples, each second visual representation is an interactive indicator that identifies the presence of pre-timeline data for the respective second parameter, wherein before interaction with the interactive indicator by a user, the value of the pre-timeline data of that interactive indicator is hidden; and after interaction with the interactive indicator by the user, the value of the pre-timeline data of the interactive indicator is visible. In this way, the second portion may effectively be an interactive button that presents the value of the pre-timeline data when a user interacts therewith. This reduces a size of the space occupied by the second portion to provide an overview of the presence of information, before providing more detailed information upon being selected. This advantageously reduces an amount of information that needs to be stored and/or displayed before a user indicates a desire to view the information.

The method may further comprise controlling a third portion of the user interface to provide a third visual representation identifying a trend in at least one second parameter before the first point in time. This provides additional useful information for an individual to make a clinical decision. The third portion may supplement the information provided in the second portion. Of course, it will be appreciated that, in some embodiments, the second visual representation itself identifies a trend in at least one second parameter before the first point in time, such that provision of a third portion is not desirable.

In some examples, each first visual representation comprises a line fitting any values of the respective first parameter. This aids in visually representing the value(s) of the first parameter(s) in a space efficient manner.

The step of obtaining, for one or more second parameters of the subject, pre-timeline data may comprise: for each of one or more first potential second parameters, processing the most recent value, before the first point in time, for the potential second parameter to predict whether or not the most recent value is reliable; and obtaining pre-timeline data for only those potential second parameters for which the most recent value, before the first point in time, is predicted to be reliable.

Thus, only reliable information may be provided at the user interface. The reliable information may, for instance, be information that is relevant or is likely to influence a state of the individual within the time period between the first point in time and the second point in time.

The step of obtaining, for one or more second parameters of the subject, pre-timeline data may comprise obtaining, for each of one or more second potential second parameters, pre-timeline data within a predetermined time of day before the first point in time.

In some examples, the step of obtaining, for one or more second parameters of the subject, pre-timeline data comprises obtaining, for each of one or more third potential second parameters, pre-timeline data of a point and/or period of time before admittance of the subject to a clinical facility and/or before performance of an intervention on the subject.

In some examples, the step of obtaining, for one or more second parameters of the subject, pre-timeline data comprises obtaining, for each of one or more fourth potential second parameters, pre-timeline data identified by a user. This allows the individual or user to control the information provided in the second portion of the user interface.

In some examples, pre-time data, for each of one or more second parameters, comprises only a single value.

In other examples, the pre-timeline data, for each of one or more second parameters, comprises a plurality of values.

It will be appreciated that there may be a plurality of second portions. Each of the plurality of second portions meets the requirements for a second portion previously herein described. This approach allows for increased flexibility in provision of data. Each second portion may contain an indicator of at least the presence of pre-timeline data for a different set of one or more second parameters and/or different pre-timeline data.

There is also provided a computer program product comprising computer program code means which, when executed on a computing device having a processing system, cause the processing system to perform all of the steps of any herein disclosed method.

There is also provided a processing system for controlling a user interface, the processing system being configured to: obtain, for one or more first parameters of the subject, timeline data containing any values of the respective first parameter between a first point in time and a second, later point in time; obtain, for one or more second parameters of the subject, pre-timeline data containing one or more values of the respective second parameter, each value of each second parameter being a value at a point in time before the first point in time; control a first portion of the user interface to provide for each first parameter, a first visual representation of the timeline data; and control a second portion of the user interface to provide, for each second parameter, a second visual representation identifying at least the presence of any pre-timeline data for that second parameter.

The quotient produced by dividing the amount of space occupied by the first portion by a first length of time, being the length of time between the first point in time and the second point in time, defines a first quotient; the quotient produced by dividing the amount of space occupied by the second portion by a second length of time, being the length of time between the furthest point in time of any value of any second parameter to the first point in time and the first point in time, defines a second quotient; and the first quotient is larger than the second quotient.

There is also provided a user interface system comprising the processing system and the user interface.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a user interface;
Fig. 2 illustrates a proposed method;
Fig. 3 illustrates a step for the proposed method;
Fig. 4 illustrates a display provided by a user interface controlled by a proposed method;
Fig. 5 illustrates another display provided by a user interface controlled by a proposed method;
Fig. 6 illustrates another display provided by a user interface controlled by a proposed method and
Fig. 7 illustrates a proposed user interface system.

### DETAILED DESCRIPTION OF EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not necessarily drawn to scale. It should also be understood that the same reference numerals are used throughout the Figures to indicate the same or similar parts.

The invention provides a mechanism for displaying timeline data and pre-timeline data of a subject. A first portion of a user interface is controlled to display the timeline data, containing data between a first and second point in time, of one or more first parameters. A second portion of the user interface is controlled to display the pre-timeline data of one or more second parameters. The amount of time between the earliest value of the pre-timeline data and first point in time, per unit space of the second portion, is less than the amount of time between the first and second points in time per unit space of the first portion.

By way of example, the temporal resolution of the first portion may be greater than the temporal resolution of the second portion, such that the first portion represents a greater amount of time per unit space than the second portion.

In the context of the present disclosure, a parameter of a subject may be any parameter that indicates a health state or condition of the subject or influences a health state or condition of the subject. In particular, each value for a parameter of a subject is a value that can be attributed to or associated with a particular point in time, in which time information for the parameter(s) should be taken into account when making a clinical decision. Thus, each value of any parameter is associated with (i.e., has) a point of time.

Examples include physiological parameters of the subject (e.g., a vital sign or other test result), indicators of any intervention performed on the subject (e.g., a medication, treatment, surgical procedure); indicators of any exercise or movement of the subject and so on.

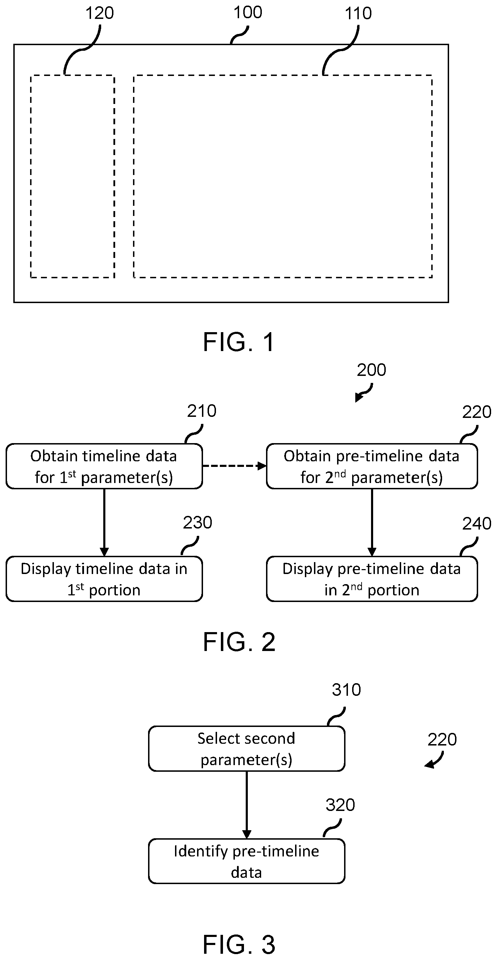

Fig. 1 illustrates a user interface 100 that has been controlled according to a proposed approach, for improved contextual understanding.

The user interface 100 may, for instance, be a screen or display (e.g., a monitor). Generally, the user interface is a device that facilitates the controllable presentation of information in visual form, i.e., a visual representation of information. Examples include liquid crystal displays (LCDs) or light emitting diode (LED) displays. Specific approaches for controlling different portions (e.g., comprising a plurality of pixels) are well established in the art.

The user interface 100 is controlled such that a first portion 110 of the user interface 100 provides a visual representation of timeline data for each of one or more first parameters of the subject. Timeline data contains values of the respective first parameter between a first point in time and a second, later point in time. Thus, timeline data comprises information about the subject relating to a particular period of time ("desired period of time").

Similarly, the user interface is controlled such that a second portion 120 of the user interface provides a visual representation of (at least) the presence of pre-timeline data for each of one or more second parameters of the subject. Pre-timeline data contains one or more values of the respective second parameter relating to one or more timepoints before the first point in time. The pre-timeline data may, for instance, include one or more (e.g., a plurality of) values of the second parameter relating to one or more timepoints between a third point in time and the first point in time (which is later than the third point in time).

The second portion 120 may be positioned to the left of the first portion 110 with respect to the perspective of a viewer of the user interface.

It will be appreciated that each value of each (first or second) parameter is associated with or has a particular point in time. The point in time may represent a time at which the value was recorded or a time at which a sample (e.g., blood sample, image, ECG result and so on) used to produce the value was performed. The precise nature will depend upon the nature of the parameter of the value.

Thus, information of the subject between a first and second point in time is visually represented in a first portion 110 of the user interface, whereas information of the subject before a first point in time is visually represented in the second portion 120.

The control of the user interface is configured such that, per unit area, the second portion represents data covering a greater period of time than the first portion. Thus, the information in the first portion may be provided at a higher temporal resolution than the information in the second portion.

It is therefore possible to define a first quotient. The first quotient is produced by dividing the amount of space (e.g., number of pixels or area) occupied by the first portion by a first length of time. The first length of time is the length of time between the first point in time and the second point in time (i.e., the length of the desired period of time).

Similarly, it is possible to define a second quotient. The second quotient is produced by dividing the amount of space occupied by the second portion by a second length of time. The second length of time is the length of time between the furthest point in time of any value of any second parameter, representing in the second portion, to the first point in time (i.e., the earliest point in time) and the first point in time.

The proposed approach for controlling a user interface therefore provides an individual with information (about a subject) pertaining to a desired period of time, whilst simultaneously providing them with at least an indication of the presence of further information before the desired period of time. The amount of time between the earliest value of the pre-timeline data and first point in time, per unit space of the second portion, is less than the amount of time between the first and second points in time per unit space of the first portion. This resolves conflicting requirements between screen space, the desire to view information within a desired period of time at a high temporal resolution and the desire to be provided with additional contextualizing information.

Thus, the amount of space occupied by the information within the desired period of time may provide more granular or higher temporal resolution than the amount of space occupied by information before the desired period of time.

Fig. 2 is a flowchart illustrating a computer-implemented method 200 for controlling a user interface. The method 200 is configured for providing an individual with information about a (clinical) subject.

The method 200 may be performed responsive to a trigger, e.g., a user input or the value of a parameter of the subject breaching a predetermined threshold. Other suitable examples for triggering the performance of method 200 will be apparent to the skilled person.

The method 200 comprises a step 210 of obtaining, for one or more first parameters of a subject, timeline data containing any values of the respective first parameter between a first point in time and a second, later point in time. The period of time between the first point in time and the second point in time may be called the desired time period or desired time window.

The one or more first parameters of the subject may be predetermined, e.g., for a particular type of user interface and/or for a particular type of clinical decision to be made using information provided at the user interface. As another example, the one or more first parameters may be identified or selected by an individual, e.g., via a user input. This facilitates user control over the information provided within the first portion of the user interface.

Similarly, the first and/or second points in time may be predetermined. For instance, the second point in time may be a most recently available point in time (e.g., a current point in time) and the first point in time may be a predetermined period of time before the second point in time (e.g., an hour before or two hours before the second point in time). As another example, the first and/or second points in time may be defined by a user of the user interface, e.g., by a user input. This advantageously allows for control over the information provided in the first portion.

There are various ways in which the desired time period can be selected.

As one example, the desired time period may be predetermined. For instance, the first point in time may be 2 or 3 hours before a current point in time and the second point in time may be the current point in time. This approach is particularly advantageous if the method 200 is triggered responsive to a value of a parameter falling into an alarm range or breaching a threshold, e.g., as it allows recent changes of parameters to be seen and reviewed.

As another example, the desired time period may be defined by a user, e.g., to reflect their desires. For example, a drop-down menu may be activatable in which different time periods can be selected (e.g., a previous: 24 hours, 8 hours, 2 hours, 1 hour, 30 min). Other approaches may allow the user to manually input the first and/or second points in time.

As another example, the method may comprise suggesting a time window. For instance, the method may suggest the use of a wider time window to include additional (potentially relevant) measurements / interventions beyond those included in a period of time identified by the clinician, e.g., to move the first point in time half an hour earlier. With a simple interaction from the individual, the time window follows this suggestion.

The method 200 also comprises a step 220 of obtaining, for one or more second parameters of the subject, pre-timeline data containing one or more values of the respective second parameter, each value of each second parameter being a value at a point in time before the first point in time.

The one or more second parameters may comprise one or more predetermined second parameters, one or more second parameters selected by a user or one or more second parameters identified using a selection process (e.g., based on the one or more first parameters and/or the first and/or second point in time). Suitable examples of a selection process are provided later in this disclosure.

The pre-timeline data of each second parameter may include all pre-timeline data of the second parameter or a sub-set (i.e., not all) of pre-timeline data of the second parameter. For instance, the pre-timeline data of each second parameter may contain only the pre-timeline data within a predetermined time period before the first point in time or only those values that meet one or more (other) criteria. Suitable examples are described later.

The method also comprises a step 230 of controlling a first portion of the user interface to provide for each first parameter, a first visual representation of the timeline data. Approaches for controlling a user interface to provide a visual representation of data are well known in the art, and are not disclosed in detail for the sake of conciseness.

By way of non-exhaustive example, the timeline data may be presented in graphical form (e.g., a line of best fit or the like), tabular form, numerical form and so on. As a preferred example, each first visual representation may comprise a line fitting any values of the respective first parameter.

The method also comprises a step 240 of controlling a second portion of the user interface to provide, for each second parameter, a second visual representation identifying at least the presence of any pre-timeline data for that second parameter.

The second visual representation may, for instance, comprise a visual representation of the pre-timeline data of the second parameter. In some examples, if the pre-timeline data is visually represented, each value in the pre-timeline data is visually represented. In some examples, the time associated with each value is also visually represented. By way of non-exhaustive example, the pre-timeline data may be presented in graphical form (e.g., a line of best fit or the like), tabular form, numerical form and so on. Of course, different instances of pre-timeline data may be presented in different formats.

In some variants in which the pre-timeline data comprises a plurality of values (each associated with a different point in time), the pre-timeline data of each second parameter may be displayed in the form of a loop. In particular, the display of the pre-timeline data in the second portion may display pre-timeline data within a moving time window. Thus, the display of pre-timeline data in the second portion may display pre-timeline data covering an P hour period (where P is any real value). The start time of the P hour period may change over time, e.g., to act as a video or loop through displaying different values of the pre-timeline data over time.

As another example, the second visual representation may comprise an interactive indicator for the second parameter, wherein (an individual) interacting with the interactive indicator (e.g., via a user interface) triggers the display of the pre-timeline data of the associated second parameter. The display of the pre-timeline data may be as previously described, although other variants are described below.

In one variant of this example, after an interaction with the interactive indicator occurs, the pre-timeline data of the second parameter may be simply displayed, e.g., overlaying a pre-existing display, e.g., in the form of a pop-up window.

In yet another variant of this example, after an interaction with the interactive indicator occurs, the first portion of the display is modified to change the points in time represented in the first portion of the user interface. In particular, consider a scenario in which the first portion displays (before interaction with the second visual representation) information of the subject between a first and second point in time. In this scenario, after an interaction with the interactive indicator occurs, the value of the first and optionally second point in times may be changed to include a period or point of time previously associated with the pre-timeline data. In some examples, the difference between the first and second points in time is maintained. In other examples, the second point in time is fixed (before and after interaction with the interactive indicator), and the first point in time is changed.

This variant effectively adjusts a period of time represented in the first portion to include the pre-timeline data, i.e., adjusting the length of the timeline to include the pre-timeline data.

As yet another example, the second visual representation may comprise an indicator of a trend of the pre-timeline data of the second parameter. By way of example, an upward pointing arrow may indicate (on average) an increase in the value(s) of the pre-timeline data for the second parameter. By way of another example, a downward pointing arrow may indicate (on average) a decrease in the value(s) of the pre-timeline data for the second parameter. In some variants, the steepness or gradient of the arrow may indicate the magnitude of the rate of change of the parameter. For instance, the steeper the arrow is, the greater the rate of change.

As yet another example, the second visual representation may comprise a visual representation of a statistical property of the pre-timeline data of the second parameter, e.g., an average (mean) or median value of the pre-timeline data.

Of course, the second visual representation may differ for different second parameters.

As previously explained, the quotient produced by dividing the amount of space occupied by the first portion by a first length of time, being the length of time between the first point in time and the second point in time, defines a first quotient; the quotient produced by dividing the amount of space occupied by the second portion by a second length of time, being the length of time between the furthest point in time of any value of any second parameter to the first point in time and the first point in time, defines a second quotient; and the first quotient is larger than the second quotient.

By way of example, the first quotient may be no less than 1.5x larger than the second quotient, e.g., no less than twice the second quotient.

The amount of space for a portion may be defined, for instance, in terms of a number of pixels of a user interface occupied by the respective portion, in terms of a total area of space occupied by the respective portion of by any other measure of space. The amount of space occupied by a portion is an amount of space reserved for the display of the relevant data. Thus, the amount of space of the first portion is an amount of space reserved for the display of the timeline data of the first parameter(s). The amount of space of the second portion is an amount of space reserved for the display of the pre-timeline data of the second parameter(s). Thus, the amount of space is the maximum possible space that could be occupied by the relevant data of the relevant parameter(s).

In some examples, the amount of space is defined as the area (e.g., in terms of pixels or other units of space such as cm²) of a rectangle that bounds or encloses the region or space reserved for a display of the relevant data. This provides a technique for easy identification of the size of a portion of a display.

In some examples, the second portion may be sub-divided into two or more sub-portions. Each sub-portion may represent a different period of time before the first point in time. The pre-timeline data for each second parameter may be positioned in the appropriate/corresponding sub-portion of the second portion, such that the visual representation of each value of the pre-timeline data is positioned within the sub-portion representing a period of time encompassing the time associated with said value.

In some such examples, the temporal resolution of each sub-portion of the second portion may be different. In particular examples, sub-portions that represent a period of time closer to the first point in time may have a higher temporal resolution than sub-portions that represent a period of time more distance from the first point in time. Preferably, there is no time gap between the periods of time represented by each sub-portion, e.g., the periods of time represented by the sub-portions abut one another.

Of course, in such examples, the temporal resolution of each sub-portion is preferably less than the temporal resolution of the first portion. Thus, each sub-portion of the second portion may represent (per unit space) a greater length of time than the first portion (per unit space).

It has previously been described how, in performing step 220, the one or more second parameters may be selected, e.g., from a pool of potential second parameters. Generally, the selected one or more second parameters may be parameters that meet one or more predetermined selection criteria. Suitable examples of selection criteria are hereafter described. The described selection criteria may be used in combination with one another and/or separately. For instance, a second parameter may need to meet at least one of one or more (e.g., a plurality of) sets of one or more selection criteria.

One example of a selection criterion for a second parameter is that the second parameter is one of the first parameters or a statistical property of one or the first parameters. This can, for instance, allow information about the first parameters before the desired period of time to be available for review by the individual or user of the user interface.

Thus, in some examples, the one or more second parameters of the subject contains only:
(all of) the first parameters of the subject; a subset (i.e., not all) of the first parameters of the subject;
and/or one or more statistical properties of one or more of the first parameters of the subject.

Another example of a selection criterion for a second parameter is that the second parameter has been identified or defined by a user. Thus, the second parameter may be a user-defined or user-identified second parameter. A user identification may be provided via a user input provided at the user interface or the like. Thus, the user may be able to select or identify one or more second parameters from a pool of potential parameters.

Another example of a selection criterion for a second parameter is that the second parameter is relevant to an intended use for the information provided at the user interface. Examples of intended use include: a clinical decision to be made, a location in which the user interface is positioned, a type, role, experience and/or interest of a clinician viewing the user interface and so on. In this approach, one or more intended uses may be associated with predetermined second parameters, e.g., in a look-up table, and only the second parameter(s) associated with one or more of the intended use(s) meet this selection criterion. The intended use may be derived from a contextual use of the proposed method or from a user input. Other suitable examples for determining an intended use of information will be readily apparent to the skilled person.

Another example of a selection criterion for a second parameter is that the second parameter is relevant for the first parameter(s). Thus, only those second parameters that may influence or affect the value of a first parameter may meet this selection criterion.

In one such example, the first parameter(s) may comprise information on one or more interventions. One selection criterion for such an example may be that the second parameter is relevant or linked to one or more of the intervention(s) represented by the first parameter(s). By way of example, if the first parameter indicates the provision of milrinone, then a selection criterion may be that the second parameter is influenced by the provision of milrinone, e .g., cardiac output (or vice versa).

Another example of a selection criterion for a second parameter may be that the number of values in the pre-timeline data of the corresponding first parameter is no more than a predetermined number N of values. The predetermined number N may, for instance, be 1, 2 or 3. This avoids provisioning of superfluous information in the form of pre-timeline data to a viewer of the user interface, e.g., as trends for the parameter may already be identifiable from the timeline data, to reduce a risk of information overload. In some examples, the predetermined number N is dependent upon whether or not the second parameter is the same as a first parameter. In particular, the predetermined number N may be lower if the second parameter is the same as a first parameter. For instance, the value of N may be 2 or 3 if the second parameter is not the same as a first parameter and may be 1 if the second parameter is the same as a first parameter.

Yet other examples of selection criteria make use of the value(s) of available data for the second parameter(s). Thus, a selection criterion for selecting a second parameter may be that the value(s) and/or time(s) associated with the value(s) of the second parameter meet one or more predetermined value criteria and/or time criteria. As previously explained, each value of any second potential parameter is associated with a particular point in time, e.g., via a timestamp or the like.

Thus, in some examples, a second parameter may be selected (e.g., for later display in the second portion) based on the value(s) of each potential second parameter and/or the time associated with the value(s) of each potential second parameter.

Hereafter described examples of selection criteria are selection criteria that make use of one or more value criteria and/or time criteria.

For instance, one example of a selection criterion for a second parameter is that a rate of change (ROC) of a value for the second parameter breaches a predetermined ROC threshold. As an example, a selection criterion may be that a rate of change of the value(s) of the second parameter within a certain time period before the desired period of time breaches the predetermined ROC threshold. The predetermined ROC threshold may be dependent upon the type or identity of the (relevant) second parameter. This approach provides an individual with information on suddenly changing second parameters.

Another example of a selection criterion for a second parameter is that at least one value for that second parameter, associated with a time before the first point in time, is still reliable and/or relevant for the desired period of time. Accordingly, it may be desirable to process for each potential second parameter, the most recent value, before the first point in time, to predict whether or not the most recent value is reliable and/or relevant.

The second parameter may therefore meet this selection criterion if the most recent value of that second parameter, before the first point in time, is predicted to be reliable and/or relevant, e.g., at the first point in time, at the second point in time or at any other point in time between the first and second points in time (e.g., midway between the first and second points in time).

Various approaches for predicting whether or not a most recent value (before the first point in time) for a second parameter is reliable and/or relevant are envisaged.

By way of example, if the most recent value is a numeric value, then the most recent value may be considered to be reliable and/or relevant if it is predicted to have not changed by more than X% by a particular time point between the first and second points in time (inclusive). X is a predetermined number, e.g., 20, 10 or 5, although other suitable examples will be apparent to the skilled person.

It is recognized that some parameters may change on short time scales, while others always stay relatively constant during a longer period of time because the underlying processes in the body are slow. Therefore, for some potential second parameters it may be useful to provide information on a past value (for making a clinical decision), even if the last value is associated with a time point significantly earlier (e.g., several hours) than the first point in time. For other parameters, a value associated with such a significantly earlier time point should not be included in a display, because there is a high possibility that the true value may have changed substantially in the meantime.

In another approach to determine whether to display pre-timeline data for a potential second parameter a look-up table may be used. In this approach the look-up table may identify, for each potential second parameter, the maximum allowable time for which the second parameter's value(s) should be displayed. It is plausible that the look-up table may define a plurality of difference maximum allowable times for each potential second parameter, each maximum allowable time being associated with a different scenario. Different scenarios may represent the occurrence of different events in the subject (e.g., the occurrence of particular interventions) or different populations of subjects.

Other approaches for defining a maximum allowable time could be used, e.g., using an algorithm or the like.

A working example of such a procedure is hereafter described for improved understanding.

In one scenario, an individual wishes to examine timeline data for one or more first parameters from a first point in time t1 (e.g., 2 hours before a current point in time) and a second point in time t2 (e.g., the current time). Thus, in this scenario, the length of the desired period of time is 2 hours. In this scenario, the latest values for three potential second parameters took place: 3 hours ago (i.e., before the current time) for a first potential second parameter, 4 hours ago for a second potential second parameter, and 8 hours ago for a third potential second parameter. Thus, the values are associated with an elapsed time at time point t1, namely: 1 hr; 2 hr; and 6 hr respectively.

Table 1 illustrates an example look-up table that defines, for each potential second parameter, a maximum allowable time in various situations (e.g., each representing the occurrence of a particular event since the time point of a value).

**TABLE 1**

| | Maximum Allowable Time (hr) | | | |
|---|---|---|---|---|
| Parameter | Default | Situation A | Situation B | Situation C |
| 1 | 4 | 1 | 0.5 | 0.5 |
| 2 | 1 | 0.25 | 0.083 | --- |
| 3 | 24 | 8 | --- | --- |

When there is no applicable situation(s), i.e. the default values in Table 1 can be used, this implies that the most recent value of the first potential second parameter is still of interest (because 1 hr (pre-timeline) is smaller than 4 hr), the most recent value of the second potential second parameter is not of interest (because 2 hr (pre-timeline) is larger than 1 hr), and the most recent value of the third potential second parameter measurement of parameter 3 is still of interest (because 6 hr (pre-timeline) is smaller than the 24 hr in the table). Therefore, the first and third potential second parameters may be selected for display.

When Situation B occurs, then only the most recent value of the third potential second parameter is still of interest and those of the first and second potential parameters should both be hidden, because they took place more than 30 and 5 minutes respectively before the start of the timeline. Thus, only the third potential second parameter is selected for display.

Another approach for determining whether a value for a potential second parameter is reliable can be used if the value is not a measurement, e.g., indicates the performance of a particular intervention. This can be achieved by determining, e.g., based on literature or known pharmacokinetic mechanisms, whether the intervention is likely to still have an effect on the subject within the desired period of time (e.g., by the first or second point in time).

By way of example only, each value for a potential second parameter (which represents the administration of a particular medication) may be associated with a known time of effect. The relevance or reliability of the value for the desired period of time can be predicted using typical times (such as half-life time) for the type of medication, based on literature or previous measurements on other subjects. Such calculations might take into account the height, weight, BMI and/or other properties of the subject. The typical times from literature and/or from previous measurements on subjects might be preprogrammed or may get updated/retrieved regularly. When connected with an infusion pump, they might also be retrieved from the medication library of the infusion pump.

As another example, a determination whether a provided medication (as indicated by a value in a potential second parameter) still has an effect on the subject during the desired time period may make use of one or more other measured parameters of the subject, e.g., the presence and/or amount of biomarkers within the blood or sweat of the subject, between the time of administration and the desired time period. Such measured parameters may be processed to determine whether or not the value(s) for the potential second parameter are still relevant or of interest for the desired period of time.

Further possible selection criteria for a second parameter are hereafter described. As before, these selection criteria are those that make use of one or more value criteria and/or time criteria.

One example of a selection criterion for a second parameter is that the second parameter has pre-timeline data within a predetermined time of day before the first point in time. Thus, this selection criterion is that the time associated with at least one value for the second parameter falls within a predetermined time of day before the first point in time.

Another example of a selection criterion for a second parameter is that the second parameter has pre-timeline data of a point and/or period of time before admittance of the subject to a clinical facility and/or before performance of an intervention on the subject (e.g., no more than Y minutes before performance of an intervention, e.g., where Y is no greater than 10 minutes). Thus, this selection criterion is that the time associated with at least one value for the second parameter falls (e.g., shortly) before admittance of the subject to a clinical facility and/or (e.g., shortly) before performance of an intervention on the subject. The intervention may be an intervention related to the potential second parameter and/or the first parameter(s), e.g., one that influences the value of the potential second parameter and/or first parameter(s). In some examples, the intervention may be identified by a user of the user interface.

This approach provides information about baseline information of the subject, e.g., to facilitate display of a normal range of the second parameters for the particular subject. This facilitates ease of comparison to values within the desired time period, e.g., to assess a normality of values within the desired time period.

Another example of a selection criterion for a second parameter is that the second parameter has pre-timeline data having one or more values that fall within one or more alarm ranges for that potential second parameter and/or outside of one or more alarm ranges. Each target range may, for instance, represent a range in a value for the second parameter is within normal or expected values. An alarm range may, for instance, represent a range in which an alarm would be generated for that second parameter.

Another example of a selection criterion for a second parameter is that the second parameter has pre-timeline data having one or more values that fall within a particular period of time before the first point in time. The particular period of time may be predetermined (e.g., 24 hours).

The particular period of time may depend upon the length of time between the first and second points in time (i.e., the length td of the desired period of time). For example, if td is small, e.g. 30 min, then an individual viewing the user interface would be looking at variations on short time scales and thus is probably interested in only recent interventions and measurements. On the other hand, if td is large, the individual viewing the user interface would probably like to get a complete picture of the subject, and it would be more appropriate to show information from pre-admission (if available) to show the subjects normal values for the parameters. Thus, the particular period of time may increase for increased lengths of the desire period of time, e.g., proportionally.

Another example of a selection criterion for a second parameter is that the second parameter is related to (e.g., provided as input to) a predictive algorithm that produces a value within the desired time period that falls within one or more predetermined ranges. For example, if HSI is 40 and therefore hemodynamic instability is expected, then any potential second parameter(s) (having one or more pre-timeline values) related to hemodynamics, such as a cardiac output may be selected.

Any combination of the selection criteria can be used in various embodiments. For instance, in some examples, a potential second parameter is only selected (for later display) if it contains a value that is predicted to still be reliable (for the desired time period) and falls within a certain range for the potential second parameter (e.g., an alarm range).

Previously described approaches relate to the selection of the second parameter(s) from a pool of potential second parameters.

It is also possible to identify the pre-timeline data for each selected second parameter, e.g., restrict the content of pre-timeline data for each selected second parameter from a larger pool or database of potential pre-timeline data.

Above disclosed selection criteria for the second parameter(s) include criteria that require at least one value and/or time (associated with a value) in the pre-timeline data of that second parameter to meet one or more value and/or time criteria.

In some examples, if one or more such value and/or time criteria are used, then only the value(s) of that second parameter that meet the one or more value criteria (if present) and/or are associated with a time that meets the one or more time criteria (if present) are obtained as the pre-timeline data for that second parameter.

In some examples, the one or more value and/or time criteria described above may be used to identify the pre-timeline data (e.g., without being used as selection criteria for the second parameter).

By way of example only, only value(s) that are still reliable and/or relevant for the desired time period may be included in the pre-timeline data for each second parameter.

As another example, only data that falls within a certain time period (e.g., a predetermined time period before the first point in time) may be included in the pre-time data for each second parameter.

In one such example, the predetermined time period is a time period during which the values of the most number of second parameters fall outside of any the corresponding alarm ranges for said second parameters (i.e., a time period during which the values of the most number of second parameters are "normal" or fall within a clinically desired or expected range). This can be readily determined by comparing the potential pre-timeline data for each second parameter to any corresponding alarm ranges or "normal"/expected ranges for said second parameter. This approach advantageously provides information that is more likely to reflect a baseline for the subject, such that deviations from this baseline can be readily ascertained. By identifying the time period during which the values of the most number (e.g., highest proportion) of second parameters falls outside any alarm ranges, unusual values for the second parameters (but which still represent the baseline for said subject) are not excluded from being considered pre-timeline data.

In another such example, the predetermined time period is defined by a user or operator for the user interface.

As another example, only data falling within a certain alarm range (e.g., for that second parameter) is included as pre-timeline data and so on. This provides useful information for identifying potential causes of alarms and parameters of risk.

Of course, it will be appreciated that the content of the pre-timeline data can be adapted, e.g., to switch between previously described examples, responsive to a user input (e.g., to change to another baseline). For instance, an individual could tap/click on the second portion, providing a visual representation of first pre-timeline data for each second parameter, to switch to second pre-timeline data for each second parameter. For instance, the display may switch, responsive to such a user input, from pre-timeline data formed from pre-admission data to displaying pre-timeline data formed from data immediately before a (particular) intervention.

In some examples, at least step 240 of method 200 is only performed if there is relevant pre-timeline data obtained in step 220. In such examples, if no second parameters and/or pre-timeline data for second parameters is identified, then the second portion is not displayed. In such examples, the method may comprise increasing the size of the first portion, e.g., not restricting the size of the first portion.

Fig. 3 illustrates one embodiment of the step 220 of obtaining, for one or more second parameters of the subject, pre-timeline data containing one or more values of the respective second parameter, each value of each second parameter being a value at a point in time before the first point in time.

In particular, in this embodiment, the step 220 comprises a sub-step 310 of selecting, from a pool of potential second parameters, one or more second parameters. A wide variety of techniques for performing sub-step 310 have been previously described.

The step 220 then performs a sub-step 320 of obtaining, for each selected second parameter, the pre-timeline data (for subsequent display).

Sub-step 320 may, for instance, comprise restricting the content of the pre-timeline data to only include the value(s) that meet certain value and/or time criteria, which may be dependent upon the nature of the selected second parameter, i.e., may differ for different second parameters. A number of examples of value and/or time criteria have been previously described.

In one example, step 320 may comprise identifying only the most recent value for the (selected) second parameter that meets the value and/or time criteria. Thus, the pre-timeline data for a second parameter may include only a single value.

As a working example, sub-step 320 may comprise restricting the pre-timeline data to only include any value(s) associated with a timepoint within a predetermined distance from the first point in time, e.g., a timepoint within a previous 24 hours.

Other examples will be apparent from previously described concepts.

Methods may be adapted to further control a third portion of the user interface. More particularly, a proposed method may further comprise controlling a third portion of the user interface to provide a third visual representation identifying a trend in at least one second parameter before the first point in time. This can supplement information provided in the second portion.

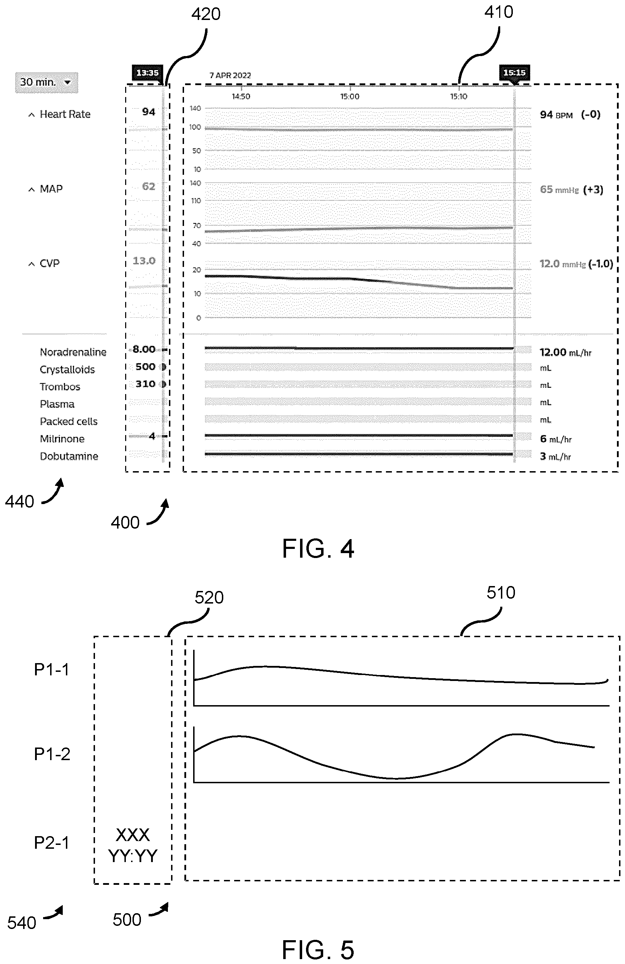

Fig. 4 illustrates an exemplary display 400 provided by a user interface controlled according to a proposed method, for improved contextual understanding.

The display 400 is conceptually divisible into a first portion 410 (providing available timeline data on first parameters) and a second portion 420 (providing available pre-timeline data for second parameters). Here, the first and second parameters are the same.

A further portion 440 of the user interface may be controlled to provide one or more labels for the first and/or second parameters. This portion may be labelled a label portion 440, and proposed methods may be appropriately adapted to include a step of controlling the user interface to provide a label portion providing labels for each first parameter and each second parameter.

Fig. 5 illustrates another exemplary display 500 provided by a user interface controlled according to a proposed method, for improved contextual understanding.

The display 500 is conceptually divisible into a first portion 510 (providing available timeline data on first parameters P1-1, P1-2) and a second portion 520 (providing available pre-timeline data for one or more second parameters P2-1, here: a single parameter). The second parameter may be relevant for one or more of the first parameters, i.e., may be a parameter that influences or is influenced by any changes in the value(s) of the one or more first parameter. As an example, if a first first parameter P1-1 is a heart rate and a second first parameter P1-2 is an SpO2, then the second parameter P2-1 may be the occurrence of a sleep apnea event. As another example, if the first first parameter P1-1 is cardiac output, then the second parameter may be an amount of Milrinone supplied to the subject.

In the illustrated example, only the most recently available value XXX of the second parameter (e.g., amount of milrinone provided) is illustrated. The time associated with the most recently available value YY: YY may also be provided.

A further portion 540 of the user interface may be controlled to provide one or more labels for the first and/or second parameters.

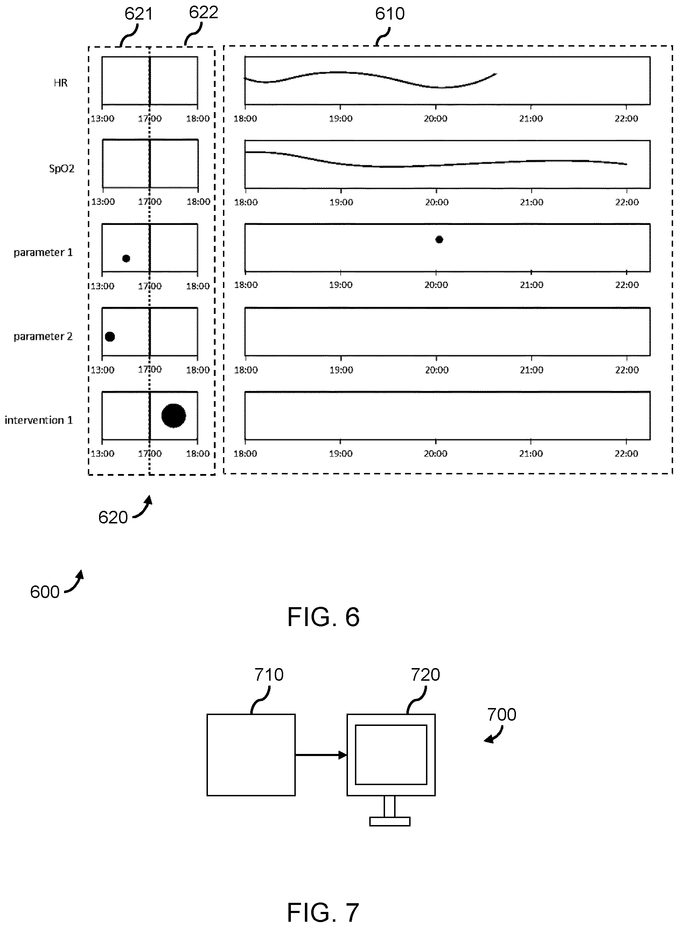

Fig. 6 illustrates another exemplary display 600 provided by a user interface controlled according to a proposed method, for improved contextual understanding.

The display 600 is again conceptually divided into a first portion 610 (providing available timeline data on first parameters) and a second portion 620 (providing available pre-timeline data for second parameters). Here, the first and second parameters are the same.

Both the first portion and the second portion illustrate available data or values within a different period of time. The temporal resolution of the first portion is greater than the second portion. Thus, per unit space, the first portion represents a greater period of time than the second portion.

In this example, the second portion 620 is sub-divided into a first sub-portion 621 and a second sub-portion 622. The temporal resolution of the first sub-portion 621 is less than the temporal resolution of the second sub-portion 622. This helps provide greater detail and granularity for pre-timeline data closer to the timeline of interest (i.e., the timeline represented in the first portion), which is usually considered to be more clinically relevant.

Fig. 7 illustrates a user interface system 700. The user interface system comprises a processing system 710 and a user interface 720. The processing system 710 is configured to perform a previously described method to control the operation of the user interface 720. The processing system 710 is itself a proposed embodiment.

The user interface 720 may be any form of visual user interface, e.g., a screen or display. The user interface 720 may, for instance, form part of a mobile device (e.g., a mobile/cellular phone, a smartphone, a tablet, a laptop, a smartwatch and so on) or other computing device (e.g., a computer or workstation). The processing system 710 may form part of the same mobile device.

The skilled person would be readily capable of developing a processing system for carrying out any herein described method. Thus, each step of the flow chart may represent a different action performed by a processing system, and may be performed by a respective module of the processing system.

Embodiments may therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method.

There is also proposed a non-transitory storage medium that stores or carries a computer program or computer code that, when executed by a processing system, causes the processing system to carry out any herein described method.

In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa. Any reference signs in the claims should not be construed as limiting the scope.

## Claims

1. A computer-implemented method for controlling a user interface, the computer-implemented method comprising:
obtaining, for one or more first parameters of a subject, timeline data containing any values of the respective first parameter between a first point in time and a second, later point in time;
obtaining, for one or more second parameters of the subject, pre-timeline data containing one or more values of the respective second parameter, each value of each second parameter being a value at a point in time before the first point in time;
controlling a first portion of the user interface to provide for each first parameter, a first visual representation of the timeline data; and
controlling a second portion of the user interface to provide, for each second parameter, a second visual representation identifying at least the presence of any pre-timeline data for that second parameter;
wherein:
the quotient produced by dividing the amount of space occupied by the first portion by a first length of time, being the length of time between the first point in time and the second point in time, defines a first quotient;
the quotient produced by dividing the amount of space occupied by the second portion by a second length of time, being the length of time between the furthest point in time of any value of any second parameter to the first point in time and the first point in time, defines a second quotient; and
the first quotient is larger than the second quotient.

2. The computer-implemented method of claim 1, wherein the one or more second parameters of the subject contains only:
the first parameters of the subject;
a subset of the first parameters of the subject; and/or
one or more statistical properties of one or more of the first parameters of the subject.

3. The computer-implemented method of claim 1 or 2, wherein the first portion of the user interface is no less than 3 times larger than the second portion of the user interface.

4. The computer-implemented method of any of claims 1 to 3, wherein the first and second portions occupy a same height of the user interface but different widths of the user interface.

5. The computer-implemented method of any of claims 1 to 4, wherein:
each second visual representation is an interactive indicator that identifies the presence of pre-timeline data for the respective second parameter;
before interaction with the interactive indicator by a user, the value of the pre-timeline data of that interactive indicator is hidden; and
after interaction with the interactive indicator by the user, the value of the pre-timeline data of the interactive indicator is visible.

6. The computer-implemented method of any of claims 1 to 5, further comprising controlling a third portion of the user interface to provide a third visual representation identifying a trend in at least one second parameter before the first point in time.

7. The computer-implemented method of any of claims 1 to 6, wherein each first visual representation comprises a line fitting any values of the respective first parameter.

8. The computer-implemented method of any of claims 1 to 7, wherein the step of obtaining, for one or more second parameters of the subject, pre-timeline data comprises:
for each of one or more first potential second parameters, processing the most recent value, before the first point in time, for the potential second parameter to predict whether or not the most recent value is reliable; and
obtaining pre-timeline data for only those potential second parameters for which the most recent value, before the first point in time, is predicted to be reliable.

9. The computer-implemented method of any of claims 1 to 8, wherein the step of obtaining, for one or more second parameters of the subject, pre-timeline data comprises obtaining, for each of one or more second potential second parameters, pre-timeline data within a predetermined time of day before the first point in time.

10. The computer-implemented method of any of claims 1 to 9, wherein the step of obtaining, for one or more second parameters of the subject, pre-timeline data comprises obtaining, for each of one or more third potential second parameters, pre-timeline data of a point and/or period of time before admittance of the subject to a clinical facility and/or before performance of an intervention on the subject.

11. The computer-implemented method of any of claims 1 to 10, wherein the step of obtaining, for one or more second parameters of the subject, pre-timeline data comprises obtaining, for each of one or more fourth potential second parameters, pre-timeline data identified by a user.

12. The computer-implemented method of any of claims 1 to 11, wherein the pre-time data, for each of one or more second parameters, comprises only a single value.

13. The computer-implemented method of any of claims 1 to 12, wherein the pre-timeline data, for each of one or more second parameters, comprises a plurality of values.

14. A computer program product comprising computer program code means which, when executed on a computing device having a processing system, cause the processing system to perform all of the steps of the method according to any of claims 1 to 13.

15. A processing system for controlling a user interface, the processing system being configured to:
obtain, for one or more first parameters of the subject, timeline data containing any values of the respective first parameter between a first point in time and a second, later point in time;
obtain, for one or more second parameters of the subject, pre-timeline data containing one or more values of the respective second parameter, each value of each second parameter being a value at a point in time before the first point in time;
control a first portion of the user interface to provide for each first parameter, a first visual representation of the timeline data; and
control a second portion of the user interface to provide, for each second parameter, a second visual representation identifying at least the presence of any pre-timeline data for that second parameter;
wherein:
the quotient produced by dividing the amount of space occupied by the first portion by a first length of time, being the length of time between the first point in time and the second point in time, defines a first quotient;
the quotient produced by dividing the amount of space occupied by the second portion by a second length of time, being the length of time between the furthest point in time of any value of any second parameter to the first point in time and the first point in time, defines a second quotient; and
the first quotient is larger than the second quotient.
